# DEMANDE DE BREVET EUROPEEN

(11) **EP 1 416 313 A1**
(43) Date de publication de la demande: **06.05.2004**
(21) Numéro de dépôt: 03292650.3
(22) Date de dépôt: 23.10.2003
(51) Int. Cl.: G02C 7/16

(54) **Lunette à multi visières**

(30) Priorité: 29.10.2002 FR 0213489
(71) Demandeur: Kassapian, Michel, 92220 Bagneux (FR)
(72) Inventeur: Kassapian, Michel, 92220 Bagneux (FR)

(57) **Abrégé**

Lunette à multiples visières aptes à protéger les yeux contre les rayons éblouissants, quels qu'en soient leurs origines et de quelque angle qu'ils pointent sur la vue, caractérisée par un ensemble de quatre visières (6-7-8-8D) reliées entre elles mais positionnables indépendamment les unes des autres, ces quatre visières étant solidarisées à une monture constituée des éléments (1-1A-2-3-3B-4-5-5C) à l'aide de coussinets élastiques (11) faisant office de clips.

## Description

L'objet de la présente invention est une lunette à multiples visières apte à protéger les yeux contre les rayons lumineux, quels qu'en soient leurs origines et de quelque angle qu'ils pointent sur la vue, assurant ainsi un champ de vision conforme au réel et non teinté par des verres colorés, de plus elle donne la possibilité de préserver les yeux contre les fortes luminosités qui peuvent incommoder la vue.

Il existe actuellement une vaste panoplie de lunettes solaires très variées dans leurs formes et les teintes de verres utilisées mais leur efficacité reste limitée aussi bien pour la protection de face que des côtés et de plus sans le bénéfice d'une vision naturelle des choses visionnées.

Il existe également des lunettes de protection utilisées dans certaines activités professionnelles et sportives, mais toutes ces lunettes quelles que soient leurs particularités, portent des verres plus ou moins teintés à l'exemple des lunettes solaires et ne peuvent avoir l'efficacité de la lunette objet de la présente invention. On peut également citer le brevet US 4 057 852 se rapporte à une visière non positionnable d'un seul tenant, non pliable pour le rangement et non muni de verre transparent.

Le brevet US 5 438 378 se rapporte à une visière destinée à être montée sur la partie supérieure de la monture d'une lunette par l'intermédiaire d'un système de clips, cette visière devant être montée sur la lunette n'est en aucune façon positionnable et qu'un tel agencement est à l'opposé de la forme de réalisation de la présente invention.

La lunette objet de cette nouveauté se distingue de tout ce qui existe en ce domaine par les dispositifs suivants : elle est constituée d'une monture classique singularisée par un pontet de pose sur le nez réglable pour un bon positionnement selon le faciès de l'usager. Cette monture sert de support à une visière opaque, concave en son côté épousant le front du porteur et d'une largeur suffisante pour faire barrage à tout rayon lumineux pointant de face quel que soit son angle de rayonnement. - Cette visière est maintenu sur la monture à l'aide de coussinets élastiques faisant office de clips fixés au verso de la visière et dans lesquels s'encastre l'élément frontal de la monture, enserrée à point voulu pour permettre la rotation de la visière par pression des doigts en vue de son positionnement à la hauteur désirée. A ce stade, la lunette préservera les yeux des rayons provenant de face mais pas contre ceux pointant par les côtés, pour parer à cet inconvénient la visière frontale sera munie sur ses deux côtés latéraux de visières mobiles reliées par des charnières permettant leurs pivotements et positionnement appropriés pour former barrage à tout éblouissement latéral. - Ces visières ailerons seront réalisées soit dans un matériau transparent plus ou moins teinté, soit totalement opaque.

L'invention a donc pour objet un dispositif de lunettes constitué d'un ensemble assurant un champ de vision naturel des choses et objets à l'abri de tout rayon éblouissant les yeux, caractérisé par l'assemblage de quatre visières dont une visière frontale opaque, de deux visières latérales opaques ou transparentes mais teintées et d'une visière transparente et teintée fixée soit au niveau de l'axe frontal soit sur le rebord avant de la visière frontale, ces quatre visières étant reliées entre elles par des charnières et ayant pour support une monture composée de deux branches d'un axe frontal reliant les deux branches à l'aide de charnières et dotée d'un pontet relié à l'axe frontal par des filins métalliques malléables, l'assemblage des quatre visières et de la monture s'effectuant à l'aide de coussinets élastiques faisant office de clips fixés au revers de la visière, la lunette ainsi constituée pouvant être portée également sur une paire de lunettes correctrice démunie du pontet et des filins et les branches raccourcies mais dotées de clips pour son accrochage sur la monture de la paire de lunette correctrice, la visière comportant un léger rehaussement sur ses trois rebords rectilignes, pour assurer une meilleure opacité aux infiltrations des rais lumineux et permettre également un repliage des visières latérales et de rebord avant de la visière frontale en vue de son rangement.

L'invention est en outre remarquable par les points suivants :
- la visière totalement opaque comprend un rebord concave pour mieux épouser le pourtour frontal de l'usager au-dessus des sourcils.
- la visière solidarisée avec l'élément de la monture à l'aide de coussinets élastiques ouverts faisant office de clips permettra le positionnement vers le sol, à l'angle souhaité de la visière.
- les visières reliées aux deux rebords latéraux de la visière pourront être positionnées séparément, et à l'angle désiré par l'usager grâce aux charnières permettant leur positionnement séparé.
- la quatrième visière est transparente ou plus ou moins teintée, amarrée sur le rebord frontal de la visière support à l'aide de charnières permettant sont positionnement à l'ange souhaité pour une protection contre les fortes luminosités et autres réverbérations indésirables.
- la monture peut être disposée sur une paire de lunettes correctrices mais s'en différenciant par le raccourcissement des branches et la suppression du pontet avec les filins relieurs mais dotée de clips pour être portée en chevauchement sur lunettes correctrices.

La lunette constituée avec ces trois visières préservera la vue contre les rayons éblouissants d'où qu'ils proviennent, mais pour parfaire cet accessoire de confort pour les yeux, il sera joint une quatrième visière transparente, plus ou moins teintée fixée au niveau de l'axe frontal ou sur le rebord avant de la visière frontale servant de support aux deux visières latérales. Cette quatrième visière complétera l'efficacité de la lunette de protection en offrant à l'utilisateur la possibilité d'atténuer toute luminosité excessive pouvant agresser les yeux sensibles.

Cette lunette à quatre volets-visières réglables répondra à toutes les attentes des ussagers pour un maximum de protection contre les agressions lumineuses.

Pour bien expliciter cette lunette, une description suivra avec référence aux dessins présentés par les deux planches ci-jointes.

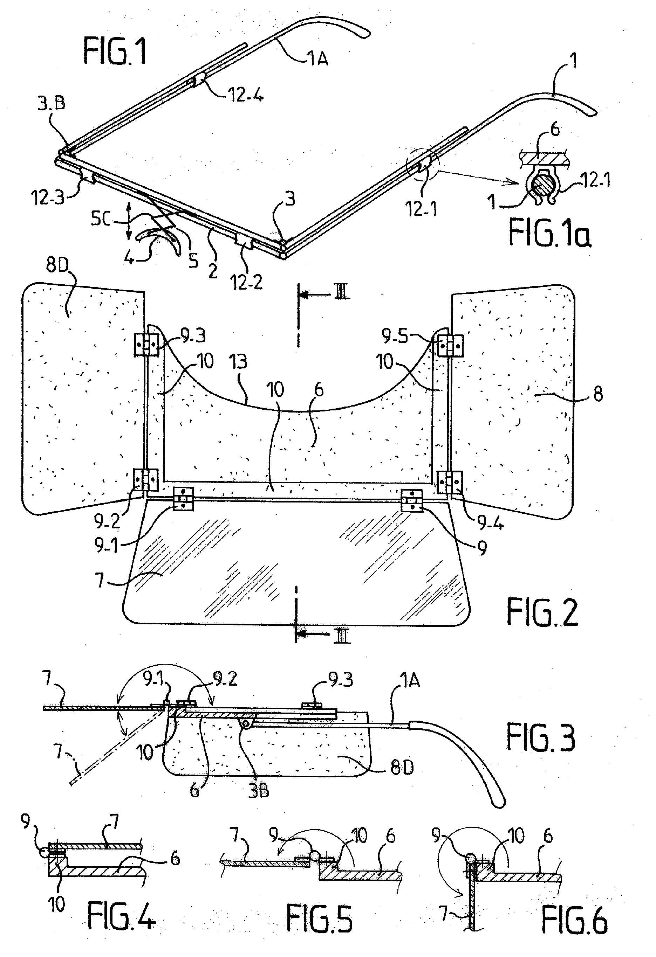

Sur la planche 1/ 2 de la figure 1 présente la monture de la lunette à visières, multiples, constituée des deux branches 1-1A reliées par l'élément frontal 2 à l'aide des charnières (3,3B) et d'un pontet 4 fixé au centre de l'élément frontal 2 par un système constitué de deux filins métalliques 5-5C pliés en croisement, donnant la possibilité d'ajustage de la lunette au-dessus des sourcils de l'usager en étirant les filins 5-5C ou au contraire en les resserrant.

La figure 2 présente un ensemble comprenant un volet visière 6 opaque, d'une largeur suffisante pour constituer une visière anti-rayons éblouissants, dont le rebord est concave pour une meilleure application au front du porteur. - Ce même volet 6 comprend un léger rehaussement 10 sur ses trois côtés rectilignes sur lesquels seront fixées les deux visières latérales 8-8D et sur le rebord frontal la visière 7 transparente est plus ou moins teintée. - Les reliages de ces trois visières 7-8-8D à la visière 6 étant effectués par des charnières 9-9.1-9.2-9.3-9.4, chacune d'entre elles pouvant être positionnée suivant l'angle d'agressivité des rayons éblouissants.

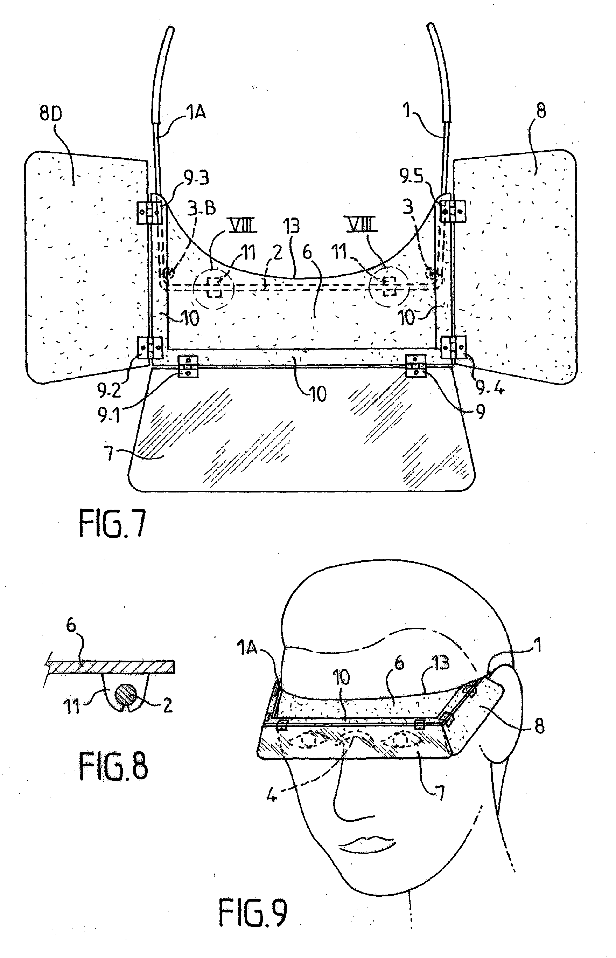

La figure 7 de la planche 2/2 présente la monture figure 1 solidarisée avec l'ensemble présenté par la figure 2. Cet assemblage est effectué à l'aide d'éléments 11 présentés par la figure 8 de la planche 2/2 faisant office de clips élastiques fixés au verso de la visière 6 dans lesquels s'encastre l'élément frontal 2 de la monture. Ce système d'assemblage par clips 11 enserrant l'élément frontal 2 permettra d'une part le pivotement de la visière 6 au plus bas vers le sol en cas de besoin et d'autre part permettra des inversions de l'ensemble des visières 6-7-8-8D par un autre ensemble de coloris différents pour une meilleure satisfaction du client.

La figure 3 présente en coupe III-III la figure 2 avec le développement à l'horizontal de la visière 7. Cette visière 7 comme les deux visières latérales 8-8D sont arrimées sur le rehaussement périphérique 10 à l'aide des charnières 9-9.1-9.2-9.3-9.4-9.5 comme présentées par la figure 2. Toutefois, l'ensemble ou partie de ces charnières pourront ne pas être utilisées pour l'assemblage par la réalisation de visières avec leurs rebords d'assemblages ouvrés à l'exemple des charnières, et solidarisés à l'aide d'une tige constituant ainsi des visières-charnières.

Les figures 4, 5 et 6 présentent en coupe, différents positionnements de la visière 7 qui sont les mêmes pour les deux visières latérales 8-8D. Ces figures 3,4, 5 et 6 démontrent également la possibilité de pliages des composantes de la lunette en vue de son rangement en étui.

Une seconde monture présentée par la figure 1 repose sur celle déjà décrite, mais se différencie de cette dernière par la suppression du pontet 4 et le raccourcissement des branches 1-1A doté de clips présentés par la figure 1A. Cette deuxième monture munie de clips 12.1-12.2-12.3-12.4-12.5 servira à utiliser la lunette par chevauchement sur les lunettes correctives.

Cette même lunette pourra également être réalisée avec les branches 1 et 1.A raccourcies, se terminant chacune en crochets dotés d'une bande élastique de longueur appropriée pour les usagers préférant le maintien de la lunette par enserrage souple autour de la tête.

Toutes les formes de réalisation représentées sur les dessins sont indicatives et non limitatives.

## Revendications

1. Dispositif de lunettes constitué d'un ensemble assurant un champ de vision naturel des choses et objets à l'abri de tout rayon éblouissant les yeux, **caractérisé par** l'assemblage de quatre visières dont une visière frontale opaque (6), de deux visières latérales opaques ou transparentes mais teintées (8-8D) et d'une visière transparente et teintée (7) fixée soit au niveau de l'axe frontal (2) soit sur le rebord avant de la visière frontale (6), ces quatre visières étant reliées entre elles par des charnières (9-9.1-9.2-9.3-9.4-9.5) et ayant pour support une monture composée de deux branches (1-1.a) d'un axe frontal (2) reliant les deux branches (1-1.a) à l'aide de charnières (3-3.b) et dotée d'un pontet (4) relié à l'axe frontal (2) par des filins métalliques malléables (5-5.c), l'assemblage des quatre visières et de la monture s'effectuant à l'aide de coussinets élastiques (11) faisant office de clips fixés au revers de la visière (6), la lunette ainsi constituée pouvant être portée également sur une paire de lunettes correctrice démunie du pontet (4) et des filins (5-5.c) et les branches (1-1.a) raccourcies mais dotées de clips (12.1-12.2-12.3-12.4) pour son accrochage sur la monture de la paire de lunette correctrice, la visière (6) comportant un léger réhaussement (10) sur ses trois rebords rectilignes, pour assurer une meilleure opacité aux infiltrations des rais lumineux et permettre également un repliage des visières latérales et de rebord avant de la visière frontale (7-8-8D) en vue de son rangement.

2. Dispositif selon la revendication 1 **caractérisé en ce que** la visière (6) totalement opaque comprend un rebord (13) concave pour mieux épouser le pourtour frontal de l'usager au dessus des sourcils.

3. Dispositif selon la revendication 1 ou 2, **caractérisé en ce que** la visière (6) solidarisée avec l'élément (2) de la monture à l'aide de coussinets élastiques ouverts faisant office de clips permettra le positionnement vers le sol, à l'angle souhaité de la visière (6).

4. Dispositif selon les revendications précédentes, **caractérisé en ce que** les visières (8-8D) reliées aux deux rebords latéraux de la visière (6) pourront être positionnées séparément, et à l'angle désiré par l'usager grâce aux charnières (9) permettant leur positionnement séparé.

5. Dispositif selon les revendications précédentes, **caractérisé en ce que** la quatrième visière (7) est transparente ou plus ou moins teintée, amarrée sur le rebord frontal de la visière support (6) à l'aide de charnières (9-9.1) permettant son positionnement à l'angle souhaité pour une protection contre les fortes luminosités et autres réverbérations indésirables.

6. Dispositif selon la revendication 1, **caractérisé en ce que** la monture peut être disposée sur une paire de lunettes correctrices mais s'en différenciant par le raccourcissement des branches (1-1A) et la suppression du pontet (4) avec les filins relieurs (5-5C) mais dotée de clips (12.1-12.2-12.3-12.4-12.5) pour être portée en chevauchement sur lunettes correctrices.
